# EUROPEAN PATENT APPLICATION

(11) **EP 0 676 204 A1**
(43) Date of publication of application: **11.10.1995**
(21) Application number: 95201369.6
(22) Date of filing: 26.07.1993
(51) Int. Cl.: A61K 31/60, A61K 31/62, A61K 31/625, A61K 9/48, A61K 9/50

(54) **Combinations of an organic nitrate and a salicylate**

(30) Priority: 30.07.1992 IE 922474
(62) Divisional of application: 93916129.5
(71) Applicant: CAL INTERNATIONAL LIMITED, Dublin 14 (IE)
(72) Inventor: Byrne, William, Mount Merrion, County Dublin (IE); Rynne, Andrew, Clane, County Kildare (IE)
(74) Representative: O'Brien, John Augustine

(57) **Abstract**

A pharmaceutical composition comprises a single dose capsule for oral administration containing an organic nitrate, preferably isosorbide 5-mononitrate and acetylsalicylic acid. A coating on one or both of the organic nitrate or acetylsalicylic acid provides a barrier between them. Portion of the organic nitrate in the capsule is in a form for immediate release and portion is in a form for slow release.

## Description

The invention relates to pharmaceutical products.

The term "organic nitrates" as used in this specification refers to pharmacologically active organic nitrate compounds which relieve, or act as prophylactic against, angina pectoris.

Organic nitrates are dilators of arterial and venous smooth muscle. The dilation action on the venous system increases the venous capacity allowing pooling of venous blood. This in turn reduces the volume of blood returning to the heart thereby lessening the strains on the heart muscle by reducing the pressure in the heart chambers (ventricles). This, in turn, reduces the oxygen requirements of the heart muscle. The dilation action on the arterial system is achieved by increasing the volume of the arterial system with consequent lower resistance to blood flow. This, in turn, reduces the work that the heart is required to do. In the coronary arteries (heart) a transient widening of the arteries (vasodilation) increases blood circulation to the heart muscle thereby increasing oxygen availability to the heart muscle.

Patients with coronary artery narrowing may suffer from angina pectoris which is usually brought on by exercise, emotion or eating. The organic nitrates by virtue of their action described above relieve the symptoms of angina pectoris.

In more detail, organic nitrates act in two ways - indirectly or directly.

Indirectly : they are smooth muscle relaxants and thus dilate both arterial and venous blood vessels. At lower doses their action is mainly on the venous system resulting in a decreased right and left ventricular filling pressure. At lower doses, however, they have little effect on the systemic (arterial) filling pressure. At higher doses, the arterial effects are more marked and decreased systemic resistance is accompanied by a reduction in blood pressure (Flaherty et al 1976). The venodilating and arterial effects of nitrates relieve ischaemia (the cause of angina, pain) by reducing determinates of myocardial oxygen demand.

Directly: they relieve ischaemia by direct action on the coronary vasculature thereby increasing intercoronary collateral flow and reversal of coronary artery spasm.

One widely used organic nitrate is isosorbide mononitrate (ISMN) which is an active metabolite of Isosorbide dinitrate (ISDN). ISMN has a high bioavailability and has a comparatively long half life (4-5 hours). This it is very suitable for prophylactic angina therapy. this is particularly so when it is presented as a sustained release formulation.

According to the invention there is provided a pharmaceutical composition comprising a single dose capsule for oral administration containing an organic nitrate and acetylsalicylic acid, a physical barrier between the organic nitrate and acetylsalicylic acid, portion of the organic nitrate in the capsule being in a form for immediate release and portion being in a form for slow release.

The organic nitrate may be an isosorbide nitrate such as isosorbide 2-mononitrate or, most preferably isosorbide 5-mononitrate.

In a preferred arrangement, the weight ratio of organic nitrate to acetylsalicylic acid is from 2:1 to 1;5, most preferably approximately 1:1.

The barrier may be a physical barrier such as a membrane between the components. The membrane may be a coating of the components in microgranular or granular presentation. The coating may be on any or all of the components within the formulation.

The invention will be more clearly understood from the following description thereof given by way of example only.

A widely used organic nitrate is Isosorbide Mono or di nitrate. Such agents act directly on the coronary arteries dilating them and thus improving the blood flow to the heart muscle and thus relieving the pain of angina pectoris. Another way that organic nitrates in general relieve the pain of angina is by reducing the requirements of the myocardium (heart muscle) for oxygen by reducing the volume of blood returning to the heart.

The pharmaceutical products of the invention are particularly for the prophylaxis of chronic stable angina pectoris. The invention provides a new combined prophylactic therapy which will deal with the pain of angina and decrease the risk of thrombosis leading to heart attack. Patients with angina pectoris have diseased coronary arteries. All patients with this degree of diseased coronary arteries are at increased risk of developing thrombosis (or clot).

Aspirin (acetylsalicylic acid) has been widely used for many years as an analgesic/anti-pyretic and anti-inflammatory agents. As such, it is a most useful drug.

In more recent years, however, it has been discovered that aspirin has a powerful antiplatelet effect. Platelets are microscopic particles within the blood that, under certain circumstances, can stick together to form a thrombus (clot). Aspirin prevents the sticking together of platelets and thus helps prevent the occurrence of heart attack or its complications.

The weight ratio of the nitrate to Aspirin may be from 2:1 to 1:5, most preferably approximately 1:1.

The component products may be separated from each other by a coating of gelatine or the like on one of the components, most preferably on the nitrate.

The effect of the pharmaceutical product of the invention is in the treatment of angina pectoris and in reducing the risk of developing myocardial infarction.

It is anticipated that, while the invention has been specifically described with reference to the combination of Isosorbide nitrate and Aspirin, it is expected that combination products of other known anti-angina agents may also be used.

Providing a nitrate and an anti platelet agent in a single dose pharmaceutical product has considerable advantages from a compliance viewpoint. If a patient is required to take a nitrate and aspirin separately there is a risk that one or other will be forgotten. It is also quicker and easier for a doctor to prescribe such a combination product.

### EXAMPLE 1

A capsule containing 80 mg of Aspirin, 15 mg of Isosorbide Mononitrate for immediate release and a slow release tablet containing 45 mg of Isosorbide Mononitrate was prepared.

A size 1 capsule was used. The ideal powder fill weight was in the region of 190 mg, containing 80 mg of Aspirin and 15 mg of Isosorbide Mononitrate. The formulation for the powder fill was :

| | | |
|---|---|---|
| A. | Aspirin | 80 mg |
| | ISMN | 15 mg |
| | Microcrystallinecellulose | 90 mg |
| | Talc | 4 mg |
| | Magnesium stearate | 1 mg |

A number of alternative formulations for a slow release tablet containing 45 mg of Isosorbide Mononitrate were made. The preferred formulation was :

| | | |
|---|---|---|
| B. | ISMN | 45.0 mg |
| | Calcium H. Phosphate | 30.0 mg |
| | Eudragit NE 40D | 15.0 mg |
| | Magnesium Stearate | 1.0 mg |
| | Water | q/s |

The ISMN was blended with Calcium H. Phosphate and the resultant mix was granulated with Eudragit. The granules were sieved using a No. 10 sieve and dried at 40°C for 6 to 8 hours. Magnesium stearate and talc were added and the mixture was blended prior to compression.

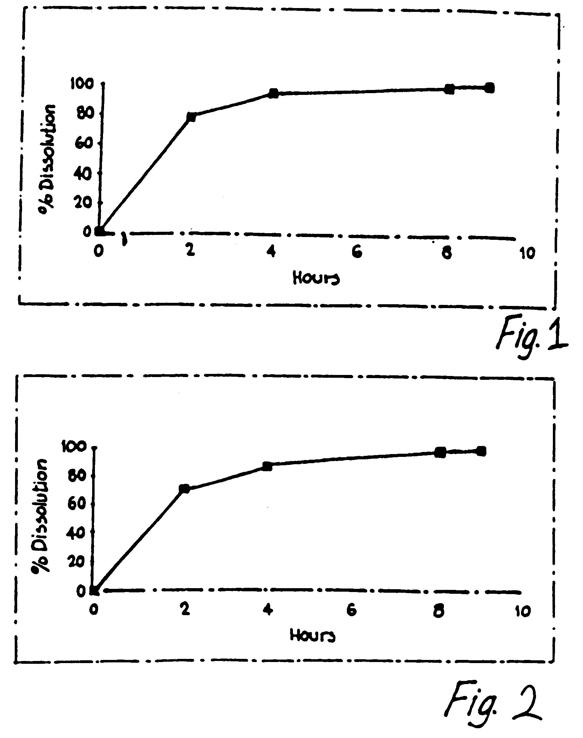

Dissolution tests of the capsule incorporating A and B yielded a good long term release profile which is plotted in Fig. 1.

### EXAMPLE 2

Example 1 was repeated except that the granules of Example 1B were further blended with Eudragit RS/PO.

The results of dissolution tests are plotted in Fig. 2.

The invention is not limited to the embodiments hereinbefore described which may be varied in detail.

## Claims

1. A pharmaceutical composition comprising a single dose capsule for oral administration containing an organic nitrate and acetylsalicylic acid, a physical barrier between the organic nitrate and acetylsalicylic acid, portion of the organic nitrate in the capsule being in a form for immediate release and portion being in a form for slow release.

2. A pharmaceutical composition as claimed in claim 1 wherein the physical barrier is in the form of a coating on one or more of the organic nitrate portion for slow release, the acetylsalicylic acid, and the organic nitrate portion for immediate release.

3. A pharmaceutical composition as claimed in claim 1 or 2 wherein the organic nitrate for slow release is in the form of a tablet in the capsule.

4. A pharmaceutical composition as claimed in claim 1, 2 or 3 wherein the organic nitrate for immediate release is in a granular or microgranular form.

5. A pharmaceutical composition as claimed in any preceding claim wherein the organic nitrate is an isosorbide mononitrate.

6. A pharmaceutical composition as claimed in claim 5 wherein the organic nitrate is isosorbide 5-mononitrate.

7. A pharmaceutical composition as claimed in any preceding claim wherein the weight ratio of organic nitrate to acetylsalicylic acid is from 2:1 to 1:5.

8. A pharmaceutical composition as claimed in claim 7 wherein the weight ratio of organic nitrate to acetylsalicylic acid is approximately 1:1.

9. A pharmaceutical composition as claimed in claim 7 or 8 wherein the weight ratio or organic nitrate to acetylsalicylic acid is approximately 3:4.
